# EUROPÄISCHE PATENTSCHRIFT

(11) **EP 1 631 252 B1**
(45) Veröffentlichungstag und Bekanntmachung des Hinweises auf die Patenterteilung: **16.09.2009**
(21) Anmeldenummer: 04734643.2
(22) Anmeldetag: 25.05.2004
(51) Int. Cl.: A61K 9/00, A61K 31/00

(54) **VERFAHREN ZUR HERSTELLUNG EINER FESTEN PHARMAZEUTISCHEN ZUSAMMENSETZUNG**
METHOD FOR PRODUCING A SOLID PHARMACEUTICAL COMPOSITION
PROCEDE DE FABRICATION D'UNE COMPOSITION PHARMACEUTIQUE SOLIDE

(30) Priorität: 05.06.2003 AT 8852003
(43) Veröffentlichungstag der Anmeldung: 08.03.2006
(73) Patentinhaber: Lannacher Heilmittel Ges.m.b.H., A-8502 Lannach (AT)
(72) Erfinder: REITER, Franz, A-8045 Graz (AT); FRISCH, Thomas, A-8054 Graz (AT)
(74) Vertreter: Nemec, Harald
(86) Internationale Anmeldenummer: PCT/AT2004/000180
(87) Internationale Veröffentlichungsnummer: WO 2004/108109

(56) Entgegenhaltungen:
- US-A- 4 205 724
- US-A- 4 482 688
- ANONYMOUS: 'Wichtige Informationen für die Einnahmme von Kaliumjodid-Tabletten nach einem Reaktorunfall' INTERNET ARTICLE, [Online] 15 Januar 2002, XP002299382 Gefunden im Internet: <URL:http://www.bmfg.gv.at/cms/site/attachm ents/4/3/4/ch0022/cms1038917442112/kj-gebra uchsinfo.pdf>
- DATABASE WPI Section Ch, Week 199325 Derwent Publications Ltd., London, GB; Class A25, AN 1993-200576 XP002299383 & JP 05 125185 A (NIPPON ZEON KK), 21. Mai 1993 (1993-05-21)

## Beschreibung

Die vorliegende Erfindung betrifft ein Verfahren zur Herstellung einer festen pharmazeutischen Zusammensetzung enthaltend ein Jodsalz, insbesondere Kaliumjodid.

Feste pharmazeutische Zusammensetzungen, welche Jodsalze, insbesondere Kaliumjodid, enthalten, werden insbesondere zu Vorsorgezwecken für den Fall einer nuklearen Bedrohung (Störfälle in Atomkraftwerken, Einsatz nuklearer Waffen) zur Verfugung gestellt.

Derzeit im Markt befindliche Zusammensetzungen, welche Kaliumjodid enthalten, enthalten kationische Polymerisate bzw. Copolymerisate auf Acrylat-/ bzw. Methacrylatbasis sowie weitere Exzipienten. Insbesondere hat sich ein Poly(butylmethacrylat,(2-dimethylaminoethyl)methacrylat, methylmethacrylat) (1:2:1), welches beispielsweise unter dem Handelsnamen "Eudragit® E 100" von der Fa. Röhm erhältlich ist, als geeignet erwiesen. Diese Substanzen werden unter anderem als Material für Schutzüberzüge um den Wirkstoff oder um die Formulierung (z.B. Tabletten) oder als Bindemittel herangezogen.

Zur Herstellung der derzeit im Markt befindlichen Zusammensetzungen wird ein Sprühgranulierungsverfahren verwendet, bei welchem das kationische Polymerisat bzw. Copolymerisat als Bindemittel in einem organischen Lösungsmittel gelöst und auf den Wirkstoff und weitere Exzipienten aufgesprüht wird.

Aus verschiedenen Gründen trachtet man jedoch danach, pharmazeutische Zubereitungen ohne die Verwendung organischer Lösungsmittel herzustellen. Es ist somit ein Ziel der vorliegenden Erfindung, ein Verfahren zur Herstellung pharmazeutischer Zubereitungen enthaltend ein Jodsalz zur Verfugung zu stellen, bei welchem der Einsatz organischer Lösemittel vermieden werden kann.

In diesem Zusammenhang hat jedoch noch ein weiterer Aspekt Bedeutung, nämlich jener der Stabilisierung des Jodsalzes. Die meistens zur Vorsorge gelagerten Jodid-Tabletten müssen über einen längeren Zeitraum haltbar sein.

Es ist bekannt, dass Jodsalze, insbesondere Kaliumjodid, bei längerer Lagerung instabil sind, was sich unter anderem in einer Verfärbung der Zubereitung (der Tablette) zeigt.

Eine weitere Aufgabe der vorliegenden Erfindung besteht somit darin, dass man Zubereitungen, insbesondere Tabletten, erhält, welche ausreichende Eigenschaften hinsichtlich der Stabilität des enthaltenen Jodsalzes aufweisen (mindestens 8 Jahre Langzeitstabilität bei 25°C / 60% R.F.), auch wenn zur Herstellung keine organischen Lösungsmittel verwendet werden.

Die Aufgaben der Erfindung werden durch ein Verfahren zur Herstellung einer pharmazeutischen Zusammensetzung enthaltend ein Jodsalz als Wirkstoff und ein kationisches Polymerisat oder Copolymerisat auf Acrylat- und/oder Methacrylatbasis, sowie gegebenenfalls weitere Exzipienten, gelöst, wobei aus dem Jodsalz, dem kationischen Polymerisat oder Copolymerisat sowie gegebenenfalls den weiteren Exzipienten in einem oder mehreren Schritten eine Mischung hergestellt wird. Das erfindungsgemäße Verfahren ist dadurch gekennzeichnet, dass das kationische Polymerisat oder Copolymerisat in fester Form und/oder in wässeriger Dispersion eingesetzt wird.

Es hat sich gezeigt, dass es möglich ist, pharmazeutische Zusammensetzungen mit zufriedenstellenden Eigenschaften auch dann herzustellen, wenn keine Lösung des kationischen Polymerisates oder Copolymerisates in einem organischen Lösungsmittel eingesetzt wird. Vielmehr hat sich überraschenderweise gezeigt, dass es möglich ist, das kationische Polymerisat oder Copolymerisat in fester Form und/oder in wässeriger Dispersion zu verarbeiten. Wenn auch die übrigen Bestandteile der Zusammensetzung (Wirkstoff(e) und Exzipienten) in fester Form und/oder in wässeriger Dispersion eingesetzt werden, ist somit eine von organischen Lösungsmitteln vollkommen freie Herstellung möglich.

Zur Herstellung der pharmazeutischen Zusammensetzung können jegliche, dem Fachmann an sich bekannte Verfahren des Mischens, Siebens etc. eingesetzt werden.

Überraschenderweise zeigen die erfindungsgemäß hergestellten Zusammensetzungen, insbesondere Tabletten, auch zufriedenstellende Eigenschaften hinsichtlich der Stabilität des Wirkstoffes, nämlich des Jodsalzes.

Es hat sich nämlich gezeigt, dass offenbar das kationische Polymerisat oder Copolymerisat eine auf das Jodsalz, insbesondere auf Kaliumjodid, stabilisierende Wirkung hat. Diese Wirkung des kationischen Polymerisates oder Copolymerisates war bislang unbekannt. Das kationische Polymerisat oder Copolymerisat wurde bislang lediglich als Hilfsstoff bezüglich der Herstellung eines Schutzüberzuges vorgeschlagen bzw. als Bindemittel verwendet.

Die stabilisierende Wirkung des kationischen Polymerisates oder Copolymerisates übertrifft die stabilisierende Wirkung von anderen als Stabilisatoren für Jodsalze bekannten Substanzen, wie z.B. Natriumascorbat, Natriumhydrogencarbonat, Natriumcitrat und Calciumcarbonat.

Bevorzugt wird im erfindungsgemäßen Verfahren als kationisches Copolymerisat ein Copolymerisat auf Basis von Dimethylaminoethylmethacrylat und neutralen Methacrylsäureestern eingesetzt. Insbesondere bevorzugt ist die Verwendung von Poly(butylmethacrylat,(2-dimethylaminoethyl)methacrylat, methylmethacrylat) (1:2:1).

Dieses Copolymer ist unter anderem unter der Bezeichnung "Eudragit® E 100" von der Firma Röhm erhältlich.

Bei einer typischen Tablettenmasse von 220 mg (enthaltend 65 mg Kaliumjodid) liegt das kationische Polymer oder Copolymer günstigerweise in einer Menge von 1 bis 5 mg vor. Allgemein sind Gewichtsanteile des kationischen Polymers oder Copolymers im Bereich von zumindest 0,1 Gew.% bis 30 Gew.%, bevorzugt 0,25 Gew.% bis 5 Gew.% günstig.

Das kationische Polymerisat oder Copolymerisat wird bevorzugt als Pulver eingesetzt.

Dabei ist insbesondere bevorzugt, dass zumindest 90% der Teilchen des Pulvers eine Teilchengröße von weniger als 0,315 mm aufweisen, und bevorzugt zumindest 80% der Teilchen eine Teilchengröße von weniger als 0,030 mm aufweisen.

Die Teilchengröße wird nach der Meßmethode gemäß Ph.Eur.2.1.4 (Ausgabe 2002) ermittelt.

Im Falle des bevorzugt verwendeten Poly(butylmethacrylat, (2-dimethylaminoethyl)methacrylat, methylmethacrylat) (1:2:1) ist unter dem Handelsnamen "Eudragit® EPO" ein Pulver im Handel erhältlich, bei welchem ca. 90% der Teilchen eine Teilchengröße von weniger als 0,030 mm aufweisen.

Bevorzugt wird im erfindungsgemäßen Verfahren nicht nur das kationische Polymerisat oder Copolymerisat in fester Form eingesetzt, sondern es werden alle Bestandteile (Wirkstoff(e) und Exzipienten) in fester Form eingesetzt.

Wird das kationische Polymerisat oder Copolymerisat in Form einer wässerigen Dispersion eingesetzt, empfiehlt sich die Verwendung von üblichen Emulgatoren und/oder Weichmachern.

Das erfindungsgemäße Verfahren ist bevorzugt dadurch gekennzeichnet, dass das Jodsalz Kaliumjodid ist.

Aufgrund der hervorragenden stabilisierenden Eigenschaften des kationischen Polymerisates oder Copolymerisates ist es möglich und bevorzugt, dass im erfindungsgemäßen Verfahren abgesehen vom kationischen Polymerisat oder Copolymerisat kein auf das Jodsalz stabilisierend wirkender Exzipient eingesetzt wird.

Die pharmazeutische Zusammensetzung wird bevorzugt in Form einer Tablette oder einer Kapsel hergestellt.

Die vorliegende Erfindung betrifft auch eine pharmazeutische Zusammensetzung, die durch das erfindungsgemäße Verfahren erhältlich ist. Die erfindungsgemäße Zusammensetzung weist bevorzugt keinerlei Anteile bzw. Rückstände an organischem Lösungsmittel auf, wie dies bei bisher bekannten Zusammensetzungen der Fall ist.

Die erfindungsgemäße Zusammensetzung ist weiters bevorzugt dadurch gekennzeichnet, dass sie abgesehen vom kationischen Polymerisat oder Copolymerisat keinen auf das Jodsalz stabilisierend wirkenden Exzipienten enthält.

In einem weiteren Aspekt betrifft die vorliegende Erfindung die Verwendung eines kationischen Polymerisates oder Copolymerisates auf Acrylat- und/oder Methacrylatbasis zur Stabilisierung von pharmazeutischen Zusammensetzungen enthaltend ein Jodsalz, insbesondere Kaliumjodid.

Besonders geeignet ist ein Copolymerisat auf Basis von Dimethylaminoethylmethacrylat und neutralen Methacrylsäureestern, insbesondere bevorzugt ist Poly(butylmethacrylat,(2-dimethylaminoethyl)methacrylat, methylmethacrylat) (1:2:1).

Die Erfindung wird im folgenden durch Beispiele näher erläutert.

### Beispiel 1 - Vergleichsbeispiel (Sprühgranulierung mit organischer Lösung)

Es wurden Tabletten entsprechend nachfolgender Rezeptur

| Tablettenkern: | |
|---|---|
| Kaliumjodid | 65,0 mg |
| Maisstärke | 53,0 mg |
| Lactose monohydrat | 80,0 mg |
| Kationisches Polymer bzw. | 1,5 mg |
| Copolymer (Eudragit®E100) | |
| (2- Propanol)* | (58,0 mg) |
| (Aceton)* | (28,0 mg) |
| Mikrokristalline Cellulose | 20,0 mg |
| Magnesiumstearat | 0,5 mg |
| | 220,0 mg |
| * wird im Zuge des Herstellungsverfahrens wieder entfernt | |

in einer Ansatzgröße von 125.000 Tabletten in folgender Weise produziert:

Der Wirkstoff, Maisstärke und Lactose wurde in einem Wirbelschichtgranulator der Type Glatt WSG-30 mit einer organischen Lösung von Eudragit®E100 (in 2-Propanol und Aceton) sprühgranuliert, sodass nach dem Sieben durch ein 1,5 mm Sieb ein homogenes, gut fließendes Granulat erhalten wurde.

Dieses wurde in einem Rhönradmischer 30 min mit Mikrokristalliner Cellulose und Magnesiumstearat gemischt.

Auf einer Rundlauftablettenpresse wurden Tabletten im Format 8 mm mit einem Gewicht von 220 mg verpresst.

### Beispiel 2 - erfindungsgemäß

Es wurden Tabletten entsprechend nachfolgender Rezeptur

| Tablettenkern: | |
|---|---|
| Kaliumjodid | 65,0 mg |
| Maisstärke | 53,0 mg |
| Lactose monohydrat | 78,5 mg |
| Kationisches Polymer bzw. | 3,0 mg |
| Copolymer (Eudragit® EPO) | |
| (H₂O)* | (123,0 mg) |
| Mikrokristalline Cellulose | 20,0 mg |
| Magnesiumstearat | 0,5 mg |
| | 220,0 mg |
| * wird im Zuge des Herstellungsverfahrens wieder entfernt | |

in einer Ansatzgröße von 3.000 Tabletten in folgender Weise produziert:

Der Wirkstoff, das kationische Copolymer, Maisstärke und Lactose wurden in einem Labor-Granulator (Type WSG) mit einer Stärkelösung granuliert, sodass nach dem Sieben durch ein 1,25 mm Sieb ein homogenes, gut fließendes Granulat erhalten wurde.

Dieses wurde in einem Turbulamischer 60 min mit Mikrokristalliner Cellulose und Maisstärke gemischt und anschließend nach Zugabe von Magnesiumstearat weitere 3 Minuten gemischt.

Auf einer Rundlauftablettenpresse wurden Tabletten im Format 8 mm mit einem Gewicht von 220 mg verpresst.

### Beispiel 3 - erfindungsgemäß

Es wurden Tabletten entsprechend nachfolgender Rezeptur

| Tablettenkem: | |
|---|---|
| Kaliumjodid | 65,0 mg |
| Maisstärke | 53,0 mg |
| Lactose monohydrat | 78,5 mg |
| Kationisches Polymer bzw. | 3,0 mg |
| Copolymer (Eudragit® EPO) | |
| Mikrokristalline Cellulose | 20,0 mg |
| Magnesiumstearat | 0,5 mg |
| | 220,0 mg |

in einer Ansatzgröße von 3.000 Tabletten in folgender Weise produziert:

Der Wirkstoff, Maisstärke, Lactose, Eudragit®EPO und Mikrokristalline Cellulose wurden in einem Turbulamischer 60 min gemischt und anschließend nach Zugabe von Magnesiumstearat weitere 3 Minuten gemischt.

Auf einer Rundlauftablettenpresse wurden Tabletten im Format 8 mm mit einem Gewicht von 220 mg verpresst.

### Beispiel 4 - erfindungsgemäß

Es wurden Tabletten entsprechend nachfolgender Rezeptur

| Tablettenkern: | |
|---|---|
| Kaliumjodid | 65,0 mg |
| Natrium-Carboxymethylstärke | 4,40 mg |
| Lactose monohydrat | 71,20 mg |
| Kationisches Polymer bzw. | 3,0 mg |
| Copolymer (Eudragit® EPO) | |
| Mikrokristalline Cellulose | 71,45 mg |
| hochdisperses Siliziumdioxid | 4,40 |
| Magnesiumstearat | 0,55 mg |
| | 220,0 mg |

in einer Ansatzgröße von 3.000 Tabletten in folgender Weise produziert:

Der Wirkstoff nebst allen Bestandteilen außer dem Magnesiumstearat wurden in einem Turbulamischer 60 min gemischt und anschließend nach Zugabe von Magnesiumstearat weitere 3 Minuten gemischt.

Auf einer Rundlauftablettenpresse wurden Tabletten im Format 8 mm mit einem Gewicht von 220 mg verpresst.

### Beispiel 5 - Vergleichsbeispiel (kein kationisches Copolymerisat, Na-Ascorbat als Stabilisator)

Es wurden Tabletten entsprechend nachfolgender Rezeptur

| Tablettenkern: | |
|---|---|
| Kaliumjodid | 65,0 mg |
| Maisstärke | 53,0 mg |
| Lactose monohydrat | 78,5 mg |
| Na-Ascorbat x 2 H₂O | 3,0 mg |
| Mikrokristalline Cellulose | 20,0 mg |
| Magnesiumstearat | 0,5 mg |
| | 220,0 mg |

in einer Ansatzgröße von 3.000 Tabletten in folgender Weise produziert:

Der Wirkstoff, Maisstärke, Lactose, Na-Ascorbat und Mikrokristalline Cellulose wurden in einem Turbulamischer 60 min gemischt und anschließend nach Zugabe von Magnesiumstearat weitere 3 Minuten gemischt.

Auf einer Rundlauftablettenpresse wurden Tabletten im Format 8 mm mit einem Gewicht von 220 mg verpresst.

Die solcherart hergestellten Tabletten wiesen bereits nach nur einer Woche eine bräunliche Verfärbung auf.

### Beispiel 6 - Vergleichsbeispiel (kein kationisches Copolymerisat, Na-Citrat als Stabilisator)

Es wurden Tabletten entsprechend nachfolgender Rezeptur

| Tablettenkern: | |
|---|---|
| Kaliumjodid | 65,0 mg |
| Maisstärke | 53,0 mg |
| Lactose monohydrat | 78,5 mg |
| Na-Citrat x H₂O | 3,0 mg |
| Mikrokristalline Cellulose | 20,0 mg |
| Magnesiumstearat | 0,5 mg |
| | 220,0 mg |

in einer Ansatzgröße von 3.000 Tabletten in folgender Weise produziert:

Der Wirkstoff, Maisstärke, Lactose, Na-Citrat und Mikrokristalline Cellulose wurden in einem Turbulamischer 60 min gemischt und anschließend nach Zugabe von Magnesiumstearat weitere 3 Minuten gemischt.

Auf einer Rundlauftablettenpresse wurden Tabletten im Format 8 mm mit einem Gewicht von 220 mg verpresst.

Die solcherart hergestellten Tabletten wiesen bereits nach nur einer Woche eine bräunliche Verfärbung auf.

### Beispiel 7 - Vergleichsbeispiel (kein kationisches Copolymerisat, Calciumcarbonat als Stabilisator)

Es wurden Tabletten entsprechend nachfolgender Rezeptur

| Tablettenkern: | |
|---|---|
| Kaliumjodid | 65,0 mg |
| Maisstärke | 53,0 mg |
| Lactose monohydrat | 78,5 mg |
| CaCO₃ | 3,0 mg |
| Mikrokristalline Cellulose | 20,0 mg |
| Magnesiumstearat | 0,5 mg |
| | 220,0 mg |

in einer Ansatzgröße von 3.000 Tabletten in folgender Weise produziert:

Der Wirkstoff, Maisstärke, Lactose, CaCO₃ und Mikrokristalline Cellulose wurden in einem Turbulamischer 60 min gemischt und anschließend nach Zugabe von Magnesiumstearat weitere 3 Minuten gemischt.

Auf einer Rundlauftablettenpresse wurden Tabletten im Format 8 mm mit einem Gewicht von 220 mg verpresst.

Die solcherart hergestellten Tabletten wiesen bereits nach nur einer Woche eine bräunliche Verfärbung auf.

## Patentansprüche

1. Verfahren zur Herstellung einer festen pharmazeutischen Zusammensetzung enthaltend ein Jodsalz als Wirkstoff und ein kationisches Polymerisat oder Copolymerisat auf Acrylat- und/oder Methacrylatbasis, sowie gegebenenfalls weitere Exzipienten, wobei aus dem Jodsalz, dem kationischen Polymerisat oder Copolymerisat sowie gegebenenfalls den weiteren Exzipienten in einem oder mehreren Schritten eine Mischung hergestellt wird, **dadurch gekennzeichnet, dass** das kationische Polymerisat oder Copolymerisat in fester Form und/oder in wässeriger Dispersion eingesetzt wird.

2. Verfahren gemäß Anspruch 1, **dadurch gekennzeichnet, dass** als kationisches Copolymerisat ein Copolymerisat auf Basis von Dimethylaminoethylmethacrylat und neutralen Methacrylsäureestern eingesetzt wird.

3. Verfahren gemäß Anspruch 2, **dadurch gekennzeichnet, dass** als kationisches Copolymerisat ein Poly(butylmethacrylat,(2-dimethylaminoethyl)methacrylat, methylmethacrylat) (1:2:1) eingesetzt wird.

4. Verfahren gemäß einem der Ansprüche 1 bis 3, **dadurch gekennzeichnet, dass** das kationische Polymerisat oder Copolymerisat als Pulver eingesetzt wird.

5. Verfahren gemäß Anspruch 4, **dadurch gekennzeichnet, dass** zumindest 90% der Teilchen des Pulvers eine Teilchengröße von weniger als 0,315 mm aufweisen, und bevorzugt zumindest 80% der Teilchen eine Teilchengröße von weniger als 0,030 mm aufweisen.

6. Verfahren gemäß einem der vorhergehenden Ansprüche, **dadurch gekennzeichnet, dass** alle Bestandteile der Zusammensetzung in fester Form eingesetzt werden.

7. Verfahren gemäß einem der vorhergehenden Ansprüche, **dadurch gekennzeichnet, dass** das Jodsalz Kaliumjodid ist.

8. Verfahren gemäß einem der vorhergehenden Ansprüche, **dadurch gekennzeichnet, dass** abgesehen vom kationischen Polymerisat oder Copolymerisat kein auf das Jodsalz stabilisierend wirkender Exzipient eingesetzt wird.

9. Verfahren gemäß einem der vorhergehenden Ansprüche, **dadurch gekennzeichnet, dass** eine pharmazeutische Zusammensetzung in Form einer Tablette oder einer Kapsel hergestellt wird.

10. Pharmazeutische Zusammensetzung, erhältlich durch das Verfahren gemäß einem der Ansprüche 1 bis 9.

11. Zusammensetzung gemäß Anspruch 10, **dadurch gekennzeichnet, dass** sie abgesehen vom kationischen Polymerisat oder Copolymerisat keinen auf das Jodsalz stabilisierend wirkenden Exzipienten enthält.

12. Verwendung eines kationischen Polymerisates oder Copolymerisates auf Acrylat- und/oder Methacrylatbasis zur Stabilisierung von pharmazeutischen Zusammensetzungen enthaltend ein Jodsalz, insbesondere Kaliumjodid.

13. Verwendung gemäß Anspruch 12, **dadurch gekennzeichnet, dass** als kationisches Copolymerisat ein Copolymerisat auf Basis von Dimethylaminoethylmethacrylat und neutralen Methacrylsäureestern eingesetzt wird.

14. Verwendung gemäß Anspruch 13, **dadurch gekennzeichnet, dass** als kationisches Copolymerisat ein Poly(butylmethacrylat,(2-dimethylaminoethyl)methacrylat, methylmethacrylat) (1:2:1) eingesetzt wird.

## Claims

1. A method for producing a solid pharmaceutical composition containing an iodine salt as an active ingredient and a cationic polymer or copolymer based on acrylate and/or methacrylate, and optionally other excipients, whereby a mixture is produced from the iodine salt, the cationic polymer or copolymer and optionally the other excipients in one or several steps, **characterized in that** the cationic polymer or copolymer is used in a solid form and/or in an aqueous dispersion.

2. A method according to claim 1, **characterized in that** a copolymer based on dimethylamino ethyl methacrylate and neutral methacrylic acid esters is used as the cationic copolymer.

3. A method according to claim 2, **characterized in that** a poly(butyl methacrylate, (2-dimethylamino ethyl)methacrylate, methyl methacrylate) (1:2:1) is used as the cationic copolymer.

4. A method according to any of claims 1 to 3, **characterized in that** the cationic polymer or copolymer is used as a powder.

5. A method according to claim 4, **characterized in that** at least 90% of the particles of the powder have a particle size of less than 0.315 mm und preferably at least 80% of the particles have a particle size of less than 0.030 mm.

6. A method according to any of the preceding claims, **characterized in that** all components of the composition are used in a solid form.

7. A method according to any of the preceding claims, **characterized in that** the iodine salt is potassium iodide.

8. A method according to any of the preceding claims, **characterized in that**, apart from the cationic polymer or copolymer, no excipient having a stabilizing effect on the iodine salt is used.

9. A method according to any of the preceding claims, **characterized in that** a pharmaceutical composition is produced in the form of a tablet or a capsule.

10. A pharmaceutical composition, obtainable by the method according to any of claims 1 to 9.

11. A composition according to claim 10, **characterized in that**, apart from the cationic polymer or copolymer, it does not contain any excipient having a stabilizing effect on the iodine salt.

12. Use of a cationic polymer or copolymer based on acrylate and/or methacrylate for stabilizing pharmaceutical compositions containing an iodine salt, in particular potassium iodide.

13. Use according to claim 12, **characterized in that** a copolymer based on dimethylamino ethyl methacrylate and neutral methacrylic acid esters is used as the cationic copolymer.

14. Use according to claim 13, **characterized in that** a poly(butyl methacrylate, (2-dimethylamino ethyl)methacrylate, methyl methacrylate) (1:2:1) is used as the cationic copolymer.

## Revendications

1. Procédé pour préparer une composition pharmaceutique solide contenant un sel d'iode en tant que substance active et un polymère ou copolymère cationique à base d'acrylate et/ou de méthacrylate, ainsi qu'éventuellement d'autres excipients, dans lequel un mélange est préparé à partir du sel d'iode, du polymère ou copolymère cationique, ainsi qu'éventuellement à partir des autres excipients en une ou plusieurs étapes, **caractérisé en ce que** le polymère ou copolymère cationique est mis en oeuvre sous forme solide et/ou dans une dispersion aqueuse.

2. Procédé selon la revendication 1, **caractérisé en ce qu'**un copolymère à base de méthacrylate de diméthylaminoéthyle et d'esters neutres de l'acide méthacrylique est mis en oeuvre en tant que copolymère cationique.

3. Procédé selon la revendication 2, **caractérisé en** en ce qu'un poly(méthacrylate de butyle, méthacrylate de 2-diméthylaminoéthyle, méthacrylate de méthyle) (1:2:1) est mis en oeuvre en tant que copolymère cationique.

4. Procédé selon l'une quelconque des revendications 1 à 3, **caractérisé en ce que** le polymère ou copolymère cationique est mis en oeuvre sous forme de poudre.

5. Procédé selon la revendication 4, **caractérisée en ce qu'**au moins 90% des particules de la poudre présentent une taille de particule inférieure à 0,315 mm et de préférence au moins 80% des particules présentent une taille de particule inférieure à 0,030 mm.

6. Procédé selon l'une quelconque des revendications précédentes, **caractérisé en ce que** tous les composants de la composition sont mis en oeuvre sous forme solide.

7. Procédé selon l'une quelconque des revendications précédentes, **caractérisé en ce que** le sel d'iode est l'iodure de potassium.

8. Procédé selon l'une quelconque des revendications précédentes, **caractérisé en ce que**, à part le polymère ou copolymère cationique, aucun excipient ayant un effet stabilisant sur le sel d'iode n'est mis en oeuvre.

9. Procédé selon l'une quelconque des revendications précédentes, **caractérisé en ce qu'**une composition pharmaceutique est préparée sous forme d'un comprimé ou d'une capsule.

10. Composition pharmaceutique pouvant être obtenue par le procédé selon l'une quelconque des revendications 1 à 9.

11. Composition selon la revendication 10, **caractérisés en ce que**, à part le polymère ou copolymère cationique, elle ne contient pas d'excipients ayant un effet stabilisant sur le sel d'iode.

12. Utilisation d'un polymère ou copolymère cationique à base d'acrylate et/ou de méthacrylate pour stabiliser les compositions pharmaceutiques contenant un sel d'iode, en particulier l'iodure de potassium.

13. Utilisation selon la revendication 12, **caractérisée en ce qu'**un copolymère à base de méthacrylate de diméthylaminoéthyle et d'esters neutres de l'acide méthacrylique est mis en oeuvre en tant que copolymère cationique.

14. Utilisation selon la revendication 13, **caractérisée en ce qu'**un un poly(méthacrylate de butyle, méthacrylate de 2-diméthylaminoéthyle, méthacrylate de méthyle) (1:2:1) est mis en oeuvre en tant que copolymère cationique.
